# EUROPEAN PATENT APPLICATION

(11) **EP 2 022 526 A1**
(43) Date of publication of application: **11.02.2009**
(21) Application number: 07015089.1
(22) Date of filing: 01.08.2007
(51) Int. Cl.: A61M 15/00

(54) **Inhaler**

(71) Applicant: Boehringer Ingelheim Pharma GmbH & Co. KG, 55216 Ingelheim (DE)
(72) Inventor: Wachtel, Herbert, 55218 Ingelheim (DE)

(57) **Abstract**

An inhaler (1) is proposed which comprises an electric or electro-mechanic or magnetic vibrating device (13). The vibrating device may be attached to or integrated into the blister (2) containing an inhalation formulation (11). Thus, deagglomeration and generating of an aerosol cloud can be improved.

## Description

The present invention relates to an inhaler according to the preamble of claim 1 and to a blister according to the preamble of claim 15.

The present invention relates to inhalers for delivery of a preferably powder-form inhalation formulation from a reservoir, in particular a blister with a blister pocket, containing the preferably premetered inhalation formulation.

The present invention relates in particular to passive inhalers, i.e. inhalers where the patient or user breathes in to generate an air stream, which entrains the inhalation formulation and forms the desired aerosol.

It is difficult to achieve optimized discharge characteristics, in particular good de-agglomeration of the powdered inhalation formulation, and/or to generate an aerosol cloud with only fine particles, preferably in the range of 2 to 10 µm, in particular 2 to 7 µm, of the inhalation formulation.

Object of the present invention is to provide an inhaler and a blister with optimized discharge characteristics.

The above object is achieved by an inhaler according to claim 1 or a blister according to claim 15. Advantageous embodiments are subject of the subclaims.

According to a first aspect of the present invention, the inhaler comprises an electric or electro-mechanic or magnetic vibrating device, which is preferably associated to the reservoir with the inhalation formulation. This enables or ensures improved de-agglomeration or loosening or aerosol generation of the inhalation formulation and, thus, optimized discharge characteristics.

The vibrating device may also be provided at or integrated into - in particular partly or completely - the reservoir of the inhaler, preferably, in a blister. Therefore, according to a second aspect of the present invention, which can be realized independently, the blister comprises an electric or electro-mechanic or magnetic vibrating device or at least part thereof.

It is possible to cause the blister pocket containing the inhalation formulation and/or the lid or any other part of portion of the blister and/or any other component of the inhaler to vibrate by means of the vibrating device. Thus, optimized de-agglomeration or loosening or aerosol generation of the inhalation formulation can be achieved resulting in optimized discharge characteristics.

In the present invention, the term "blister" has to be understood preferably in the usual sense in particular such that a pre-formed base is covered by a lid. However, the term "blister" can also be understood in a broader sense to include other, preferably similar reservoirs, in particular with at least partly flexible or elastic or thin components or walls, according to the present invention.

Further aspects, features, properties and advantages of the present invention are described in the claims and the subsequent description of preferred embodiments, with reference to the drawing. There are shown in:
- Fig. 1: a schematic sectional view of an inhaler according to a first embodiment of the present invention;
- Fig. 2: a schematic view of details and possible arrangements of a vibrating device of the inhaler according to Fig. 1;
- Fig. 3: a schematic view of a blister with an associated vibrating device according to a second embodiment of the present invention;
- Fig. 4: a schematic view of a blister with an associated vibrating device according to a third embodiment of the present invention;
- Fig. 5: a schematic view of a blister with an associated vibrating device according to a fourth embodiment of the present invention;
- Fig. 6: a schematic view of a blister with an associated vibrating device according to a fifth embodiment of the present invention;
- Fig. 7: a schematic view of a blister with an associated vibrating device according to a sixth embodiment of the present invention;
- Fig. 8: a schematic view of a backside of a blister with an associated vibrating device according to a seventh embodiment of the present invention;
- Fig. 9: another schematic view of the seventh embodiment according Fig. 8;
- Fig. 10 a: schematic view of a blister with an associated vibrating device according to an eighth embodiment of the present invention; and
- Fig. 11: a schematic view of a blister with an associated vibrating device according to a ninth embodiment of the present invention.

In the Figures, the same reference numbers are used for identical or similar parts, even if a repeated description is omitted. In particular identical or corresponding advantages and properties then also result or may be achieved.

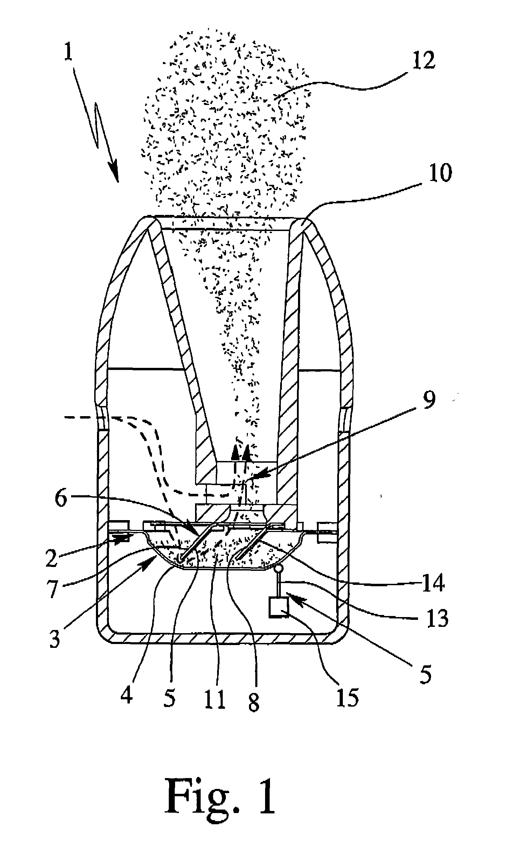

Fig. 1 shows in a schematic sectional representation an inhaler 1 according to a first embodiment of the present invention. Preferably, the inhaler 1 is portable and/or is hand-held.

The inhaler 1 serves to deliver a preferably powdered inhalation formulation from a reservoir, in particular a blister 2 with a blister pocket 3 containing directly a dose of the preferably loose inhalation formulation. The inhalation formulation is preferably pre-metered.

The blister 2 having the blister pocket 3 forms the preferred reservoir. Therefore, it is referred in the following often only to the blister 2 and/or blister pocket 3. However, the present invention, description and explanation shall apply preferably to other kinds of reservoirs in a similar manner as well.

The present inhaler 1 is preferably designed for single use only. However, it also possible to design the inhaler 1 for multiple use, in particular to provide the inhaler 1 with an exchangeable reservoir or with a reservoir or blister 2 with multiple, preferably pre-metered doses of the inhalation formulation, in particular multiple blister pockets 3.

Fig. 1 shows the inhaler 1 with already opened blister pocket 3 during delivery (dispensing) of the inhalation formulation. In the or non-activated state, the blister 2 / blister pocket 3 is still closed. In particular hermetically sealed.

The blister 2 or blister pocket 3 preferably consists of or comprises a base 4 covered by a lid 5. In particular, the base 4 forms or comprises at least one curved or bowl-like receiving space or depression covered by the lid 5 for sealingly receiving the inhalation formulation. However, other designs are possible.

The present inhaler 1 preferably comprises a piercing member 6 for opening, in particular puncturing or cutting or rupturing, the blister 2 or its blister pocket 3, in particular the lid 5. However, other opening devices or methods could alternatively or additionally be used, e.g. peeling of the lid 5.

In the shown embodiment, the piercing member 6 preferably comprises at least one, here two piercing elements 7, 8 for opening or puncturing the blister pocket 3, in particular its lid 5 as shown in Fig. 1.

In particular, the first piercing element 7 serves the form a first blister opening (inlet opening) in the lid 5. The second piercing element 8 forms a separate, second blister opening (outlet opening) in the lid 5.

The piercing member 6 or opened blister pocket 3 is in fluid connection or connectable with a feeding path 9 of the inhaler 1 to deliver or dispense the inhalation formulation via a mouthpiece 10 or the like.

During or for inhalation a patient or user, not shown, places the mouthpiece 10 in his mouth and breathes in. Thus, an air stream of ambient air is sucked in and passed or guided through the opened blister pocket 3 such that loose powder 11 (forming the inhalation formulation and being schematically shown in Fig. 1) is entrained in the air and dispensed with the sucked-in ambient air as an aerosol cloud 12 via the feeding path 9 and mouthpiece 10, as schematically shown Fig. 1.

In the present embodiment, preferably air is used to dispense the inhalation formulation. However, any other fluid or gas could be used. Preferably, the term "air" or "air stream" has to be understood in a broad sense including other gas as well.

In the present embodiment, the inhaler 1 is preferably a passive inhaler, i.e. the inhalation formulation is dispensed by breathing in of a patient or user (not shown). However, the present description and invention preferably also apply to active inhalers, i.e. when a means for pressurizing gas or any other fluid (e,g, a compressor, an air pump, liquefied or compressed gas or the like) is provided to actively dispense the inhalation formulation.

The present invention deals in particular with the dispensing of an inhalation formulation in powder-form by means of a gas stream. The following description focuses on this scenario, However, the present invention may also be used to generate an aerosol by means of any other fluid as medium for dispensing the inhalation formulation, e.g. a liquid dissolving and/or dispensing a dry inhalation formulation, Alternatively or additionally, a liquid inhalation formulation can be dispensed by means of gas and/or a liquid as feeding medium. Then, the present explanations shall apply preferably accordingly.

According to the present invention, an electric or electro-mechanic or magnetic vibrating device 13 is provided. The vibrating device 13 is in particular associated to the reservoir / blister 2 / blister pocket 3. In particular, the vibrating device 13 generates vibrations or hits so that at least part of the blister pocket 2, in particular at least a portion of the base 4 and/or lid 5, and/or the feeding path 9 vibrate. This facilitates or supports or enables or ensures and improved de-agglomeration, loosening or aerosol generating of the inhalation formulation.

However, the vibrating device 13 can alternatively or additionally also cause the piercing member 6 or any other element extending into the preferably opened blister pocket 3 and/or any other component of the inhaler 1 to vibrate.

Fig. 1 shows only a very schematic view how the vibrating device 13 may be associated to the blister 2 or blister pocket 3.

The vibrating device 13 comprises preferably a contact, hammer or vibrating element 14 (hereinafter called vibrating element) and a drive 15 driving the vibrating element 14 as shown in Fig. 1. Here, the vibrating element 14 contacts the blister pocket 3 or its base 4 from outside.

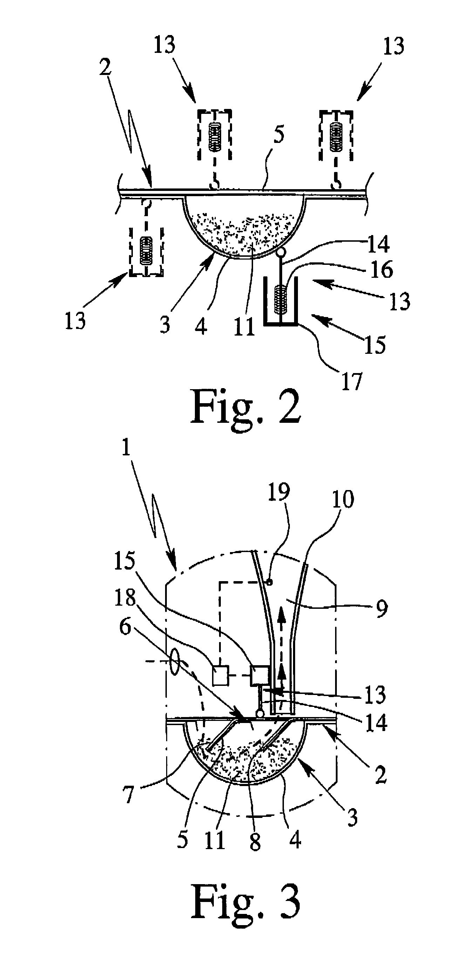

Fig. 2 shows in a very schematic view a preferred design of the vibrating device 13. In particular, the vibrating device 13 or drive 15 comprises a plunger coil or moving coil 16 that can oscillate relative to an associated, preferably bell-shaped magnet 17. Preferably the plunger coil 16 directly moves or actuates the vibrating element 14. This construction or other constructions, in particular as used in microphones, are particularly suitable to generate vibrations and/or to achieve the desired vibration frequencies as described later.

In the first embodiment, the vibrating device 13 contacts the blister 2, in particular the blister pocket 3, more precisely, the base 4 in the region of the blister pocket 3, from the outside. However, other arrangements are possible.

Fig. 2 shows in dashed lines other possible arrangements of the vibrating device 13, For example, the vibrating device 13 or its vibrating element 14 can contact the blister 2 from its backside, i,e, the base 4, adjacent to the blister pocket 3.

The vibrating device 13 or vibrating element 14 can contact alternatively or additionally also the front side of the blister 2, i.e, the lid 5, optionally in the area where the lid 5 covers the blister pocket 3, i.e. is not in contact with the base 4, or where the lid 5 is in contact with the base 4 as shown in Fig. 2 by the two upper possible placements of the vibrating device 13.

Fig. 3 shows in a schematic view the inhaler 1 according to a second embodiment of the present invention. Here, the vibrating device 13 can cause at least part of the piercing member 6 to vibrate.

In the second embodiment, the inhaler 1 comprises preferably a control device 18, preferably an integrated circuit, a microcontroller or the like, for controlling the vibrating device 13. Most preferably, the vibrating device 13 is electrically or electronically controlled and/or operated.

The operation of the vibrating device 13 can be controlled in different aspects, e.g. with regard to time, duration, intensity, amplitude, frequency or the like of the generated or caused vibration or hits. Further a sensor 19 may be provided so that the vibration generation may be controlled also depending on additional information, such as air pressure in the mouthpiece 10, flow rate of inhalation air, actuation of a button or the like by the patient or user (not shown), or the like.

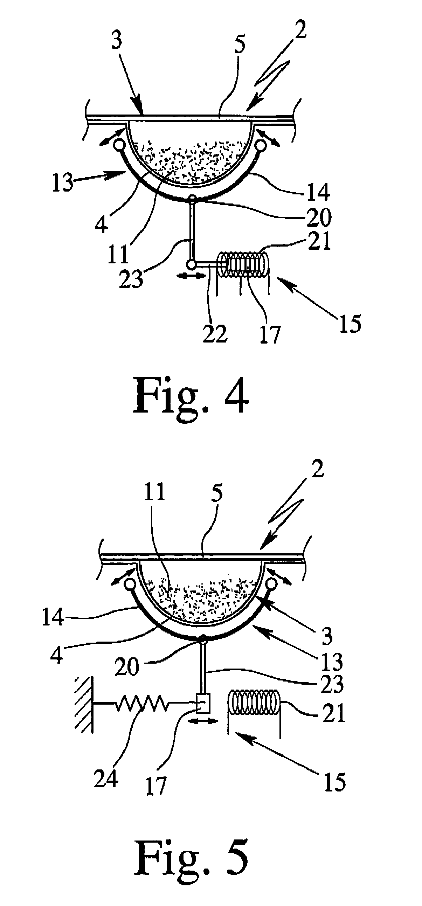

Fig. 4 shows in a schematic side view a third embodiment. Here, the vibrating element 14 contacts or vibrates the blister pocket 3 at two different or opposite sides or portions and/or in two different or opposite directions. Preferably, the vibrating element 14 is a two-armed lever pivotable around an axis 20 in the third embodiment. For example, the drive 15 comprises a hollow coil 21 in which the magnet 17 or a magnetic element (anchor) can be electrically moved back and forth. This movement is transmitted in particular by means of a suitable drive train, e.g. an actuating element 22 connected with to magnet 17 or magnetic element and an actuating arm 23 pivotally connected with the actuating element 22 and radially connected with the vibrating element 14 as shown in Fig. 4.

Fig. 5 shows a fourth embodiment which is similar to the third embodiment. Here, the magnet 17 or a magnetic element (anchor) is moved by the electromagnet / coil 21 against the force of a spring 24 back and forth. Thus, a preferably perodic attraction of the magnet 17 / magnetic element is sufficient. This arrangement is particularly suitable for discrete hits to cause the blister 2 to or its base 4 to vibrate.

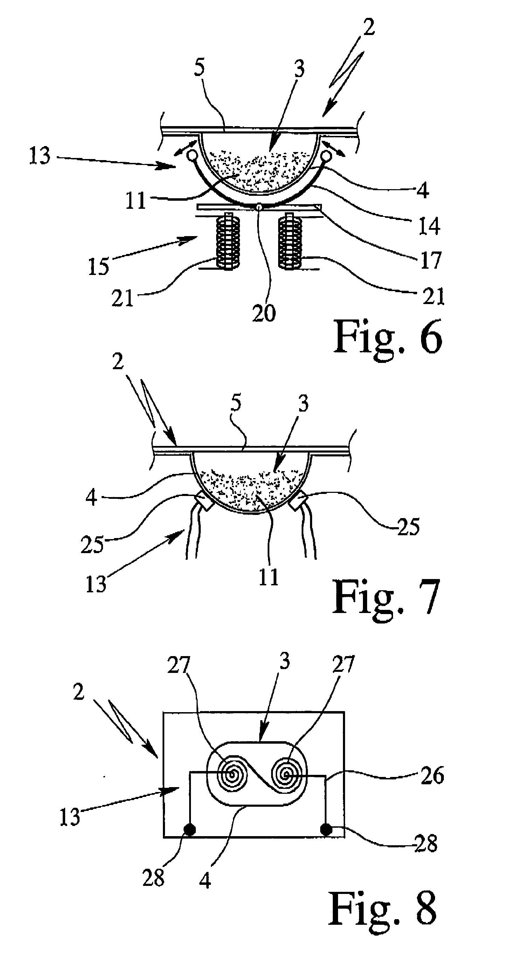

Fig. 6 shows a fifth embodiment, which is similar to the third and fourth embodiment. Here two electromagnets or coils 21 alternatively attract the preferably bar like magnet 17 or magnetic element / anchor so that the vibrating element 14 is pivoted back and forth.

Fig. 7 shows a sixth embodiment. Here, the vibrating device 13 comprises at least one, preferably two or more piezo elements 25 (preferably stacks of piezoelectric crystals or he like) that can cause the blister pocket 3, in particular its base 4, to vibrate either indirectly - e.g. via a respective mechanic, drive train or the like (not shown) - or directly (by respective contact with the base 4 or any other part of the reservoir or any other part that shall vibrate). In particular, it is possible and provided that the piezo element(s) 25 is/are directly attached to or integrated into the part that shall vibrate, such as the blister 2, blister pocket 3, base 4 or lid 5. In the shown embodiment, the piezo elements 25 are preferably located on opposite portions or sides of the blister pocket 3 or base 4. However, other arrangements are also possible.

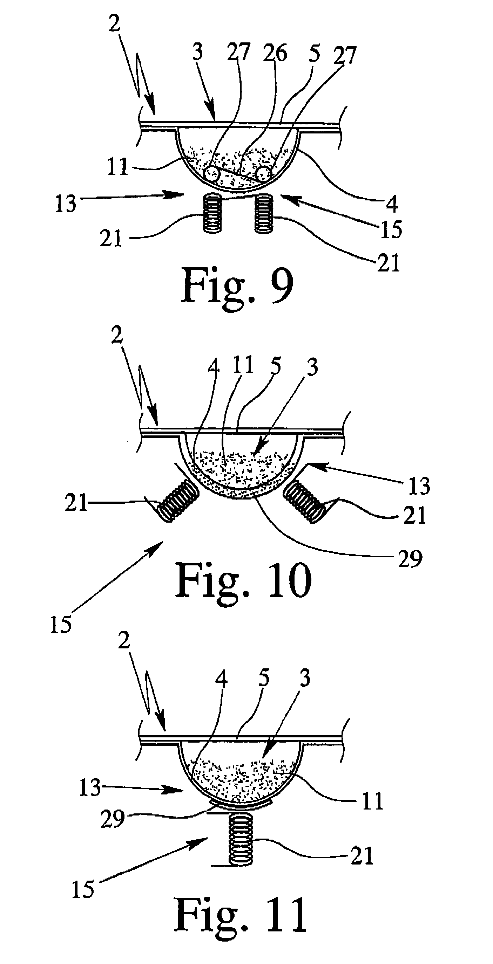

Fig. 8 and 9 show a seventh embodiment of the present invention. Here, a conductor 26 is formed directly on or integrated into the blister 2, in particular the base 4 in the area of the blister pocket 3.

The conductor 26 may form one or multiple coils 27 or other conducting arrangements. If necessary, the conductor 26 or blister 2 may comprise or form associated electric contacts 28 as shown in Fig. 8. The conductor 26 may be attached, e.g. glued, to the blister 2 or its base 4 or formed by etching or by any other suitable process.

The conductor 26 and/or coils 27 cooperate preferably with one or two preferably stationary electromagnets or coils 21 as shown in Fig. 9. By respectively controlling the current flowing through the conductor 26 / coil(s) 27 and through the coils 21, the blister pocket 3 or base 4 can be caused to vibrate, in particular if the preferred pairs of one coil 21 and one adjacent coil 27 attract alternatively or simultaneously.

It has to be noted that instead of the stationary coils 21 also stationary magnets or magnetic elements arranged near by the coils 27 could be used. Then, the movement and vibrating of the base 4 is only controlled by the electric current flowing through the conductor 26 / coil(s) 27.

Fig. 10 shows an eighth embodiment. Here, magnetic material 29 is attached to or integrated into the wall or part of portion, in particular base 4, that shall vibrate. The magnetic material may be attracted by one or multiple electromagnets or coils 21 as schematically shown in Fig. 10. Thus, it is possible to vibrate the base 4 or any other flexible part with the magnetic material 29 as desired, e.g. by alternatively attracting portions with the magnetic material 29 or by periodically attracting portions with the magnetic material 29.

Alternatively the base 4 or portion, that shall vibrate, can be made of magnetic material.

Fig. 11 shows a ninth embodiment, which is very similar to the eighth embodiment. Here, the magnetic material 29 forms a coating or cover on the blister 2, in particular on the base 4, or any other portion or component that shall vibrate.

The frequency of vibration is preferably adapted to the respective component or part that shall vibrate.

In particular, the vibration frequency is adapted to the respective inhaler 1, respective reservoir, to the respective inhalation formulation, to the amount and/or consistence of the inhalation formulation and/or the like.

According to one preferred aspect, the vibration frequency of the vibrating device 13 or a harmonic frequency thereof corresponds essentially - at least temporarily - to the resonance frequency of the part or portion that shall vibrate, such as the blister 2, blister pocket 3, lid 5, base 4, piercing member 6, piercing element 7 or 8, feeding path 9 and/or mouthpiece 10.

Most preferably, the vibration frequency of the vibrating device 13 is between 1 and 500 kHz, in particular about between 10 and 200 kHz.

According to another or additional aspect of the present invention, the vibration frequency of the vibrating device 13 varies, in particular wobbles, during a delivery operation or inhalation process.

The vibrating device 13 may operate feedback-controlled, e.g. as a hammer break or hammer interrupter.

Additionally or alternatively, the vibrating device 13 or its vibrating element 14 may act like a hammer that transmits one pulse or hit or multiple pulses or hits by respective strikes or hits to the respective component. Thus, the component will vibrate with its resonance frequency. The hammering frequency can be considerably lower, e.g. in the range of 5 Hz and 500 Hz.

It has to be noted that features of the different embodiments and/or the different embodiments may be combined as desired and/or used for other inhalers or reservoirs as well.

### List of reference numbers

- 1: inhaler
- 2: blister
- 3: blister pocket
- 4: base
- 5: lid
- 6: piercing member
- 7: first piercing element
- 8: second piercing element
- 9: feeding path
- 10: mouthpiece
- 11: powder
- 12: aerosol cloud
- 13: vibrating device
- 14: vibrating element
- 15: drive
- 16: plunger coil
- 17: magnet
- 18: control device
- 19: sensor
- 20: axis
- 21: stationary coil
- 22: actuating element
- 23: actuating arm
- 24: spring
- 25: piezo element
- 26: conductor
- 27: coil
- 28: contact
- 29: magnetic material

## Claims

1. Inhaler (1) for delivery of an inhalation formulation from a reservoir, preferably a blister (2) with a blister pocket (3), containing the inhalation formulation, comprising:
a mouthpiece (10); and
a feeding path (9) fluidically connected or connectable to the reservoir;
the inhaler (1) being designed such that a stream of a fluid, in particular gas, preferably air, can discharge a dose of the inhalation formulation from the reservoir and through the feeding path (9) to deliver it via the mouthpiece (10),
**characterized in**
**that** the inhaler (1) or reservoir comprises an electric or electro-mechanic or magnetic vibrating device (13).

2. Inhaler according to claim 1, **characterized in that** the inhaler (1) is designed such that the vibrating device (13) can cause at least part of the reservoir to vibrate.

3. Inhaler according to claim 1 or 2, **characterized in that** the inhaler (1) is designed such that the vibrating device (13) can cause at least part of the feeding path (9) to vibrate.

4. Inhaler according to one of the previous claims, **characterized in that** the inhaler (1) comprises a piercing member (6) for opening, in particular puncturing, a lid (5) of the reservoir or blister pocket (3).

5. Inhaler according to claim 4, **characterized in that** the inhaler (1) is designed such that the vibrating device (13) can cause at least part of the piercing member (6) or any other element extending into the reservoir to vibrate.

6. Inhaler according to claim 4 or 5, **characterized in that** the vibration frequency of the vibrating device (13) or a harmonic frequency thereof corresponds essentially - at least temporarily - to the resonance frequency of at least part of the piercing member (6) or any other element extending into the reservoir.

7. Inhaler according to one of the previous claims, **characterized in that** the vibration frequency of the vibrating device (13) or a harmonic frequency thereof corresponds essentially - at least temporarily - to the resonance frequency of at least part of the reservoir, such as a base (4) or Lid (5).

8. Inhaler according to one of the previous claims, **characterized in that** the vibration frequency of the vibrating device (13) is between 1 and 500 kHz, preferably about between 10 and 200 kHz.

9. Inhaler according to one of the previous claims, **characterized in that** the inhaler (1) is designed such that the vibration frequency of the vibrating device (13) varies, in particular wobbles, during a delivery operation of the inhaler (1).

10. Inhaler according to one of the previous claims, **characterized in that** the vibrating device (13) or at least a part thereof, such as an electric coil (27) or a magnetic, electric or piezoelectric element (25, 26, 29), is attached to or integrated into the reservoir.

11. Inhaler according to one of the previous claims, **characterized in that** the vibrating device (13) comprises an electromagnet or a coil (21) or a plunger coil (16), and an associated magnet (17) or magnetic element or anchor.

12. Inhaler according to one of the previous claims, **characterized in that** the vibrating device (13) comprises a piezoelectric element (25).

13. Inhaler according to one of the previous claims, **characterized in that** the vibrating device (13) comprises a hammer or vibrating element (14) for contacting the reservoir preferably from the outside and/or on different portions or opposite sides to transmit a hit or vibration to the reservoir or any other component or portion of the inhaler (1) that shall vibrate.

14. Inhaler according to one of the previous claims, **characterized in that** the inhalation formulation is powder (11).

15. Blister (2) for delivery of an inhalation formulation, with a blister pocket (3) containing the inhalation formulation,
**characterized in**
**that** the blister (2) comprises an electric or electro-mechanic or magnetic vibrating device (13) or at least part thereof.

16. Inhaler according to claim 15, **characterized in that** the blister comprises a lid (5) and a curved, preferably bowl-like base (4) covered by the lid (5).

17. Blister according to claim 15 or 16, **characterized in that** the vibration frequency of the vibrating device (13) or a harmonic frequency thereof corresponds essentially - at least temporarily - to the resonance frequency of at least part of the reservoir, such as a lid (5) or base (4).

18. Blister according to one of claims 15 to 17, **characterized in that** the vibration frequency of the vibrating device (13) is between 1 and 500 kHz, preferably about between 10 and 200 kHz.

19. Blister according to one of claims 15 to 18, **characterized in that** the vibrating device (13) or at least a part thereof, such as an electric coil (27) or a magnetic, electric or piezoelectric element (25, 26,29), is attached to or integrated into the blister (2), in particular into the lid (5) and/or base (4) thereof.

20. Blister according to one of claims 15 to 19, **characterized in that** the inhalation formulation is powder (11).
